# EUROPEAN PATENT APPLICATION

(11) **EP 3 838 258 A1**
(43) Date of publication of application: **23.06.2021**
(21) Application number: 19217163.5
(22) Date of filing: 17.12.2019
(51) Int. Cl.: A61K 9/00, A61K 9/08

(54) **PARENTERAL NUTRITION SOLUTION COMPRISING A SELENIUM SOURCE**

(71) Applicant: Baxter International Inc., Deerfield, IL 60015 (US); Baxter Healthcare SA, 8152 Glattpark (Opfikon) (CH)
(72) Inventor: DUPONT, Caroline, 1050 Ixelles (BE); BOUREZG, Zouaoui, 1420 Braine l Alleud (BE)
(74) Representative: Hertin und Partner Rechts- und Patentanwälte PartG mbB

(57) **Abstract**

The invention relates to a medical product for preventing or correcting selenium deficiency in a patient comprising a solution provided in a flexible container characterized in that the solution comprises at least one of a selenate salt and/or a seleno-amino acid, such as selenomethionine or selenocysteine.

## Description

The invention relates to the field of clinical nutrition, corresponding medical products and nutritional solutions.

The invention relates to a medical product for preventing or correcting selenium deficiency in a patient comprising a solution provided in a flexible container characterized in that the solution comprises at least one of a selenate salt and/or a seleno-amino acid, such as selenomethionine or selenocysteine.

In embodiments of the invention, the solution of the medical product of the invention comprises at least one additional trace element. The solution may be contained in one chamber of a multi chamber container having at least two, at least three, at least four, at least five or at least six chambers. Furthermore, the invention relates to a method of preparing a medical product of the invention.

### BACKGROUND OF THE INVENTION

Parenteral Nutrition aims to supply nutrients to patients by venous access. Nutrients are composed of macronutrients (lipids, amino acid or protein and dextrose or carbohydrates), micronutrients (vitamins and trace elements) and electrolytes.

Parenteral nutrition, such as in the form of one or more solutions, can be provided in the form of flexible bags, either in the form of single flexible bags containing glucose, amino acids or lipids with or without electrolytes, which can be mixed together prior administration, or multi chamber flexible bags providing separated macronutrients and electrolytes, in a ready to use format. The bags are typically made of a synthetic or plastic material, for example materials such as polypropylene (PP), polyethylene (PE), ethylene vinyl alcohol (EVOH), ethylene-vinyl acetate (EVA) and all possible copolymers, essentially any synthetic material suitable for containing the components to be administered. In embodiments, the material is oxygen-impermeable.

In the state of the art, micronutrients are typically added to nutrition bags directly before administration. For this purpose, vitamins can be provided in glass vials in the form of lyophilizates or solutions to be reconstituted and/or mixed into the nutrition/infusion bags. Trace elements are also provided in glass vials or polypropylene ampules meant to be mixed into infusion bags prior to administration.

Prior to usage, meaning administration to patient, the micronutrients are sometimes added in the mixture or macronutrients via an injection port of the container or bag (septum) or are added via a Y-connector to the infusion line. This process takes time and several handling steps increasing the risk of errors or contamination.

To avoid these potential problems, products have been developed that already contain some trace elements in nutrition multi-chamber bags, such as, for example, Pediaven, a parenteral nutrition binary solution intended for infants, children and adolescents, which contains trace elements in the glucose chamber. However, it has been reported that the trace element selenium, provided as selenium dioxide in the product, is absent in the finished product potentially due to degradation, as announced in July 2014 (http://www.pharmacovigilance-tours.fr/490.html). Another product, Elneopa, from Otsuka Pharmaceuticals, contains certain trace elements in a small dedicated chamber as part of a multi-chamber bag. However, this product does not contain selenium.

It is known in the art that selenium, iodine and copper are difficult to include in nutrition bags, as they can undergo chemical reactions, especially under extreme conditions such as a heat sterilization step and during the storage period [for example, Allwood et al. Compatibility and Stability of Additives in Parenteral Nutrition Admixtures. Nutrition 1998, Vol. 14, No. 9, pp. 697-706; Eisenberg et al. Stability of selenium sources reviewed. Feedstuffs, June 18, 2012].

Furthermore, in various formulation studies, when attempting to introduce trace elements into nutrition multi-chamber bags, serious stability issues have been experienced, in particular the loss of selenium has been observed. This may be due to the fact that selenium in the form of sodium selenite (and selenious acid) is prone to adsorption, for example to plastic materials or iron oxides; can be reduced into metallic selenium in the presence of reducing agents like ascorbic acid; can be reduced into hydrogen selenide, which is a volatile substance; and/or can be transformed into selenious dioxide at low pH, which is also a volatile substance under certain conditions. Furthermore, nutritional solutions comprising selenate salts are unknown in the state of the art.

In addition to selenium, iodine, fluoride and copper also showed stability issues during formulation trials. Copper is a reactive entity and can catalyse various chemical reactions and it is known that it can precipitate. Iodide can be reduced into iodine, which is potentially volatile. Furthermore, fluoride showed a decreasing concentration over time.

Accordingly, to date there is no ready-made, sterilized medical product available that comprises a solution for parenteral administration to a patient in need thereof, comprising selenium, which is stable over a prolonged period of time. Specifically, no such ready-made product provided in a flexible bag has been made available so far. Selenium, and potentially also other trace elements, must be manually added to the ready-made solutions shortly before administration. This process is associated with a significant risk of making mistakes, for example with respect to the amount of the trace elements, or of introducing contamination to the sterilized ready-made products. Contaminations can include potential infectious agents, which represent a notorious and serious risk factor to patients in hospitals, especially already compromised patients in need of receiving nutritional solutions e.g. via parenteral administration.

In light of the prior art, there remains a significant need to develop ready-to-use, sterilized medical products for intravenous or parenteral administration to be used for preventing or correcting selenium deficiency in a patient. Specifically, what is missing are products which are straightforward and easy to use, whilst avoiding additional mixing steps. Due to the instability of selenium in solution, such products are not readily available.

### SUMMARY OF THE INVENTION

In light of the prior art the technical problem underlying the present invention is to provide a pre-prepared and ready-to-use medical product for preventing or correcting selenium deficiency in a patient, comprising a solution with selenium provided in a flexible container, preferably in a flexible container which is made from an oxygen-impermeable material.

This problem is solved by the features of the independent claims. Preferred embodiments of the present invention are provided by the dependent claims.

The invention therefore relates to a medical product for preventing or correcting selenium deficiency in a patient comprising a solution provided in a flexible container characterized in that the solution comprises at least one of a selenate salt and/or a seleno-amino acid, such as selenomethionine or selenocysteine, selenomethionine or selenocysteine.

In embodiemnts, the invention relates to a medical product for preventing or correcting selenium deficiency in a patient comprising a solution provided in a flexible container characterized in that the solution comprises at least one of a selenate salt, selenomethionine or selenocysteine.

In embodiments of the invention, the solution of the medical product of the invention comprises a selenate salt. In some embodiments, the solution of the medical product of the invention comprises selenomethionine. In some embodiments, the solution of the medical product of the invention comprises selenocysteine.

It was entirely surprising that selenate salt, selenomethionine or selenocysteine are stable in a solution provided in a flexible container of a medical product of the invention. This is particularly unexpected since it is generally expected that these selenium containing compounds are instable due to degradation and adsorption processes. Also, reduction processes are believed to lead to instability of selenium containing compounds. In this context, vitamins have been reported to be sensitive to oxygen when stored in sealed containers, such as in sealed flexible bags.

However, the present invention is based on the unexpected finding that specifically selenate salts, such as sodium selenate or potassium selenate, but also selenomethionine or selenocysteine are stable in solutions provided in flexible containers of a medical nutritional product.

In embodiments of the medical product of the invention, the selenate salt, selenomethionine and/or selenocysteine are stable in the solution for at least three months when stored at a temperature of between 1 and 50 °C. It was unexpected that comparative experiments could demonstrate that the listed selenium containing compounds were stabilized in solution during various temperatures tested in the range of 1 to 50 °C for prolonged periods, such as at least three months.

In embodiments, the selenate salt, selenomethionine and/or selenocysteine are stable in the solution for at least three months when stored at up to 40 °C. Furthermore, in embodiments the selenate salt, selenomethionine and/or selenocysteine are stable in the solution for at least six months when stored at up to 40 °C.

In further embodiments of the invention, the selenate salt, selenomethionine and/or selenocysteine are stable in the solution for at least 6 months, preferably 12 months, more preferably 18 months, most preferably 24 months.

In embodiments of the invention, the selenate salt, selenomethionine and/or selenocysteine are stable in the solution for at least 6 months, preferably 12 months, more preferably 18 months, most preferably 24 months, at a temperature of up to 30 °C.

In further embodiments of the invention, the selenate salt, selenomethionine and/or selenocysteine are stable in the solution for at least 6 months, preferably 12 months, more preferably 18 months, most preferably 24 months, at a temperature of from about 18°C to 25°C.

In further embodiments of the invention, the selenate salt, selenomethionine and/or selenocysteine are stable in the solution for at least 6 months, preferably 12 months, more preferably 18 months, most preferably 24 months, at a regular storage temperature, including for example, but without limitation thereto, of temperatures of room temperature, varying from 15-30 °C, more preferably 18-25 °C, or storage in refrigerated conditions, such as from 1 to 10 °C, preferably 2 to 8, or 3 to 7 °C.

In embodiments of the invention, the solution of the medical product is a sterilized solution. In some embodiments, the solution of the medical product of the invention may be sterile.

It is an important advantage that the solution of the present invention can be sterilized after preparation and packaging into a flexible bag that may be sealed airtight and liquid tight without a significant loss of the trace elements provided therein such as in particular selenate salts, selenomethionine and/or selenocysteine. In embodiments, the solution of the invention undergoes terminal heat sterilization after preparation of the solution. Sterilization may occur prior or after filling of the solution into the flexible bag of the medical product of the invention.

Sterilization of the product may occur by a terminal heat sterilization process but also by using terminal filtration, gamma irradiation or any other sterilization technique. Also, the solution of the medical product of the invention may be filled into the flexible bag by an aseptic filling process ensuring that no contamination of the essentially sterile solution occurs during filling and before sealing of the flexible bag. In some embodiments, the solution may have been sterilized, but is not necessarily sterile. For example, a sterilization treatment may have been conducted, but an absolute sterility not achieved.

In the context of the invention the terms flexible bag and flexible container may be used interchangeably.

It is a great advantage of the invention that selenium provided as selenate salts, selenomethionine and/or selenocysteine is stable in the context of the medical product of the invention since no complicated technical measures including complicated technical instrumentation or manipulation of the solution have to be established for stabilization.

In embodiments, the solution of the medical product comprises a selenate salt, preferably selected from the group consisting of sodium selenate, potassium selenate, barium selenate and ammonium selenate, more preferably selected from sodium selenate and potassium selenate. The use of selenate salts and in particular sodium selenate and potassium selenate in the context of the present invention are particularly advantageous since it was found that these salts are very stable in solutions, in particular at neutral and acidic pH. This was entirely surprising, since other sources of selenium, such as sodium selenite, have been reported and observed to be instable in acidic or neutral solutions that do not comprise further features.

The medical product according to any one of the preceding claims, wherein the solution has an acidic pH, preferably in the range of from 1 to 4, more preferably from 2 to 3.5, more preferably from about 2.5 to 3.2. It is a particular advantage of the solution of the present invention that selenate salts, selenomethionine and/or selenocysteine are stable also in solutions with an acidic pH and not only at about neutral pH in the range of 7 to 7.5. The selenium in the solution of the invention is in particular also stable at acidic pH, such as a pH in the range of 1 - 4, 1.5 - 3.5, 1.8 - 3.4, 2 - 3.3, 2.1 - 3.2, 2.2 - 3.1, 2.3 - 3.0, 2.4 - 2.9, 2.5 - 2.8 and 2.6 - 2.7. The indicated ranges include the noted end-values. Ranges comprising any combination of disclosed end-values are considered as embodiments of the invention.

The stability at such acidic pH conditions is important, in particular if the solution also includes other trace elements that may not be stable at neutral pH, but only under acidic conditions. This is in particular the case for iodide (I), which has been reported to be more stable in solutions with acidic pH.

However, this may also be the case for nutritional solutions comprising one or more of the trace elements copper (Cu), zinc (Zn), iron (Fe), manganese (Mn), chromium (Cr), fluoride (F) and molybdenum (Mo). Accordingly, it is an important advantage of embodiments of the medical product of the invention that the solution with acidic pH can stabilize not only selenium, but also other trace elements in the solution for extended storage periods.

In embodiments of the invention, the solution comprises an acid, preferably an organic acid selected from the group comprising malic acid, tartaric acid, citric acid, maleic acid, fumaric acid, more preferably malic acid, wherein the concentration of the organic acid is preferably in the range of from 50 mM to 400 mM, preferably from 190 mM to 220 mM, and more preferably about 200 mM.

In another embodiment, the solution comprises an acid, preferably an organic acid selected from the group consisting of malic acid, tartaric acid, citric acid, maleic acid, fumaric acid, more preferably malic acid, wherein the concentration of the organic acid is preferably in the range of from 50 mM to 400 mM, preferably from 100 mM to 200 mM.

In another specific embodiment, the solution comprises malic acid. In embodiments the solution comprises malic acid at a concentration in the range of from 50 mM to 400 mM, preferably from 190 mM to 220 mM, such as, for example, 140 to 180 mM or 160 mM to 200 mM. The use of malic acid in the context of a nutritional medical product is particularly advantageous since it is an organic acid that naturally occurs in fruits, such as apples, apricots, blackberries, blueberries, cherries, grapes, peaches and others and is particularly well tolerated by human subjects when administered in the context of a nutritional product.

The acid concentration in the solution of the medical product of the invention can be in any range that leads to a suitable pH value for the desired application. Depending on the acid, a different concentration may be required to achieve for example an acidic pH value in the range of 1 - 4. A person skilled in the art can choose and adjust appropriately a suitable acid concentration for a preferred embodiment comprising a solution of a given pH and a specific acid.

In embodiments of the medical product of the invention, the solution does not comprise macronutrients. Preferably those selected from the group comprising carbohydrates, proteins and lipids, and wherein preferably the solution does not comprise any other nutrients.

In further embodiments the solution does not comprise carbohydrates. In embodiments the solution does not comprise proteins or amino acids, besides optionally those comprising selenium. In embodiments the solution does not comprise lipids. In embodiments the solution may not comprise electrolytes. In embodiments the solution does not comprise vitamins. In embodiments the solution does not comprise other trace elements other than selenium. In embodiments the solution only contains trace elements. In embodiments the solution contains selenium as the only trace element. In embodiments the solution contains selenium as the only nutrient. In embodiments the solution contains selenium as the only nutrient, besides water (in case water is considered as a nutrient).

In embodiments, the solution of the medical product of the invention comprises at least one additional trace element, preferably selected from the group comprising zinc, iron, copper, manganese, chromium, iodine, fluoride and molybdenum.

In embodiments, the solution of the medical product of the invention comprises at least one additional trace element, preferably selected from the group comprising zinc, iron, manganese and/or copper.

In embodiments, the solution of the medical product of the invention comprises at least one additional trace element, preferably selected from the group consisting of zinc, iron, copper, manganese, chromium, iodine, fluoride and molybdenum. In embodiments, the solution of the medical product of the invention comprises at least one additional trace element, preferably selected from the group consisting of zinc, iron, manganese and/or copper.

In embodiments, the solution of the medical product of the invention comprises as additional trace elements at least zinc, iron, manganese and copper. In embodiments, the solution of the medical product of the invention comprises as additional trace elements at least zinc, iron, manganese, copper, chromium, iodine, fluoride and molybdenum.

In embodiments, the solution of the medical product of the invention comprises as additional trace elements at least zinc, iron, manganese, copper and chromium. In embodiments, the solution of the medical product of the invention comprises as additional trace elements at least zinc, iron, manganese, copper and iodine. In embodiments, the solution of the medical product of the invention comprises as additional trace elements at least zinc, iron, manganese, copper and fluoride. In embodiments, the solution of the medical product of the invention comprises as additional trace elements at least zinc, iron, manganese, copper and molybdenum.

In embodiments, the solution of the medical product of the invention comprises as additional trace elements at least zinc, iron, manganese, copper, chromium and iodine. In embodiments, the solution of the medical product of the invention comprises as additional trace elements at least zinc, iron, manganese, copper, chromium and fluoride. In embodiments, the solution of the medical product of the invention comprises as additional trace elements at least zinc, iron, manganese, copper, chromium and molybdenum.

In embodiments, the solution of the medical product of the invention comprises as additional trace elements at least zinc, iron, manganese, copper, iodine and fluoride. In embodiments, the solution of the medical product of the invention comprises as additional trace elements at least zinc, iron, manganese, copper, iodine and molybdenum.

In embodiments, the solution of the medical product of the invention comprises as additional trace elements at least zinc, iron, manganese, copper, fluoride and molybdenum.

In embodiments, the solution of the medical product of the invention comprises as additional trace elements at least zinc, iron, manganese, copper, chromium, iodine and fluoride. In embodiments, the solution of the medical product of the invention comprises as additional trace elements at least zinc, iron, manganese, copper, chromium, iodine and molybdenum. In embodiments, the solution of the medical product of the invention comprises as additional trace elements at least zinc, iron, manganese, copper, chromium, fluoride and molybdenum. In embodiments, the solution of the medical product of the invention comprises as additional trace elements at least zinc, iron, manganese, copper, iodine, fluoride and molybdenum.

In the context of the invention, zinc can be provided as any suitable compound, such as a salt, such as preferably gluconate, chloride or sulfate. In the context of the invention, iron can be provided as any suitable compound, such as a salt, such as preferably gluconate, chloride or sulfate. In the context of the invention, copper can be provided as any suitable compound, such as a salt, such as preferably gluconate, chloride or sulfate. In the context of the invention, manganese can be provided as any suitable compound, such as a salt, such as preferably gluconate, chloride or sulfate. In the context of the invention, chromium can be provided as any suitable compound, such as a salt, such as preferably gluconate, chloride or sulfate. In the context of the invention, iodine can be provided as any suitable compound, such as a salt, such as preferably potassium or sodium iodide. In the context of the invention, fluoride can be provided as any suitable compound, such as a salt, such as preferably potassium or sodium fluoride. In the context of the invention, molybdenum can be provided as any suitable compound, such as a salt, such as preferably gluconate, chloride or sulfate.

As additional nutritional elements in addition to selenium, the medical product of the invention can comprise at least one of the following nutrients: Carbohydrate, such as mainly dextrose, dextrins, polyols; amino acids, peptides and/or proteins; electrolytes, such as sodium, magnesium, potassium, calcium and/or phosphate; lipids, such as olive oil, soybean oil, fish oil, algae oil, rapseed oil, structured lipids of long, medium and short chains fatty acids; vitamins, such as vitamin A, B1, B2, B3, B5, B6, B8, B9, B12, C, D, E and/or K.

Furthermore, embodiments of the invention relate to a medical product, wherein the solution is administered parenterally. In further embodiments, the solution of the medical product of the invention is administered or consumed orally. In embodiments, the solution can be administered enterally.

In the context of the present invention, the solution of the claimed medical product can comprise a selenate salt, selenomethionine and/or selenocysteine in an amount corresponding to 10-200 µg, preferably 40-100 µg, more preferably about 55 - 75 µg, most preferably about 60-70 µg of selenium.

In embodiments, the amount of the selenium in the solution of the medical product corresponds to a recommended daily dose of selenium, wherein a daily dose may be quantified as the mass of selenium to be administered to a patient per day, for example µg of selenium per patient per day (µg/patient/day). In embodiments, the amount of selenium in the solution of the product may be in the range of 1 - 500, 2 - 450, 4 - 400, 6 - 350, 7 - 300, 8 - 270, 9 - 235, 10 - 200, 15 - 190, 20 - 180, 25 - 170, 30 - 160, 35 - 150, 40 - 140, 45 - 130, 50 - 120, 52 - 115, 54 - 110, 56 - 105, 58 - 100, 60 - 95, 62 - 90, 64 - 88, 66 - 86, 68 - 84, 70 - 82, 72 - 80, 74 - 78 or 76 µg selenium. The indicated ranges include the noted end-values. Ranges comprising any combination of disclosed end-values are considered as embodiments of the invention.

In the context of the solution of the invention, selenium is provided as a selenate salt, selenomethionine and/or selenocysteine. Accordingly, for example, when 70 µg of selenium are provided in form of sodium selenate (Na₂SeO₄), which has a molar mass 188.947 g/mol, wherein selenium has a molar mass of 78.97 g/mol, this corresponds to about 167,5 µg sodium selenate.

Accordingly, when 70 µg of selenium are provided in form of selenomethionine (SeMet or C₅H₁₁NO₂Se), which has a molar mass 196.106 g/mol, wherein selenium has a molar mass of 78.97 g/mol, this corresponds to about 173,7 µg selenomethionine.

Furthermore, when 70 µg of selenium are provided in form of selenocysteine (Se-Cys or C₃H₇NO₂Se), which has a molar mass 168.065 g/mol, wherein selenium has a molar mass of 78.97 g/mol, this corresponds to about 148.9 µg selenocysteine.

As evident from these examples, a skilled person is able to calculate the amount of a compound comprising selenium in a solution of a medical product of the invention on the basis of the molar mass of said compound and the amount of selenium present in the solution. In the same way, it is possible to calculate the amounts of compounds comprising other trace elements on the basis of the molar mass of the respective trace element and compound comprising said trace element.

In embodiments, the medical product of the invention represents a daily nutritional dose for a patient. Accordingly, the amounts of the components of the product, such as the selenium in the solution of the product, are calculated and provided in order to cover a daily dose of a patent for said compound. Depending of the volume of the solution of the product and the amount of selenium, the concentration of selenium in the solution can be calculated.

In embodiments of the medical product of the invention, the solution in the flexible container has a volume of about 25 ml. In such an embodiment, the amount of selenium in the solution can be 70 µg, and the resulting concentration of selenium can be 2.8 µg/ml (or 2.8 mg/l). Se-concentration ranges can be calculated on the basis of the amounts of Se disclosed herein. Preferred Se-concentrations of the invention are in the range of 0.28 - 28 mg/l. However, the Se-concentration may also be in the range of 0.1 - 100 mg/l, 0.2 - 90, 0.3 - 85, 0.4- 80, 0.5 - 75, 0.6 - 70, 0.7 - 65, 0.8 - 60, 0.9 - 55, 1 - 50, 1.2 - 48, 1.4 - 46- 1.6 - 44, 1.8 - 42, 2 - 40, 2.2 - 38, 2.4 - 36, 2.6 - 34, 2.8 - 32, 3 - 30, 3.5 - 29, 4 - 28, 4.5 - 27, 5 - 26, 5.5 - 25, 6 - 24, 6.5 - 23, 7 - 22, 7.5 - 21, 8 - 20, 9 - 19, 10 - 18, 11 - 17, 12 - 16, 13 - 15 or 14 mg/ml. The indicated ranges include the noted end-values. Ranges comprising any combination of disclosed end-values are considered as embodiments of the invention.

The following ***Table 1*** indicates suitable ranges and preferred amounts for adults of trace elements that can be comprised by the medical product and in particular by the solution of the medical product of the invention. Ranges and amounts for pediatrics may be different and can be adapted according to the applicable recommendations. Such suitable ranges can be updated from time to time, so any preferred amounts of trace elements that can be comprised in a medical product according to the invention may be adapted to recommendations currently in force.

**Table 1. Suitable amounts and ranges of amounts of trace elements that can be used in the context of the invention. Indicated ranges are based on different ranges recommended in the art. The amounts refer to a medical product of the invention that provides one daily dose for one patient. The indicated ranges include the noted end-values. Ranges comprising any combination of disclosed end-values are considered as embodiments of the invention.**

| Trace element | ESPEN 2009 [17] | DGEM 2009 [24] | ASPEN 2012 [13] | AuSPEN 2014 [40] | Consensus paper 2019 [41] | Preferred amount #1 | Preferred amount #2 |
|---|---|---|---|---|---|---|---|
| Zinc (µg) | 2500-5000 | 2500-4000 | 3000-5000 | 3200-6500 | 2500-6500 | 5000 | 5000 |
| Iron (µg) | 1000-1200 | 1000-1500 | - | 1100 | 1000-1200 | 1000 | NA |
| Copper (µg) | 300-500 | 300-1200 | 300-500 | 317-508 | 300-610 | 300 | 300 |
| Manganese (µg) | 200-300 | 200-800 | 55 | 55 | 55-100 | 55 | 55 |
| Chromium (µg) | 10-15 | 10-20 | 10-15 | 10-15 | 10-15 | 10 (or NA) | (or NA) |
| Selenium (µg) | 20-60 | 20-80 | 60-100 | 60-100 | 20-100 | 70 | 60 |
| Iodine (µg) | 100 | - | - | 126 | 70-150 | 100 | NA |
| Fluoride (µg) | 1000 | - | - | - | - | 1000 | NA |
| Molybdenum (µg) | 20 | - | - | 19 | No recommenddation | 20 | NA |

Table 2 indicates suitable concentration ranges for adults of trace elements that can be comprised by the solution of the medical product of the invention. Ranges for pediatrics may be different and can be adapted according to the applicable recommendations. Such suitable ranges can be updated from time to time, so any preferred amounts of trace elements that can be comprised in a medical product according to the invention may be adapted to recommendations currently in force.

**Table 2. Suitable concentrations and concentration ranges of trace elements (TE) that can be used in the context of the solution of the medical product of the invention. Indicated ranges refer to the preferred daily dose of a patient as listed in Table 1 and volumes of 2.5 to 250 ml of the solution.**

| TE | Method LOQ mg/L | Chemical Form | Chemical form; Solubility in water | Batch 1 concentration (mg/L; 1dd/250ml) | Batch 2 concentration (mg/L; 1dd/25ml) | Batch 3 concentration (mg/L; 1dd/2.5ml |
|---|---|---|---|---|---|---|
| Zn | 3.5 | ZnCl₂ | 4.32 kg/L | 20 | 200 | 2000 |
| Cu | 0.350 | CuCl₂, 2H2O | 706g/L | 1.2 | 12 | 120 |

| TE | Method LOQ mg/L | Chemical Form | Chemical form; Solubility in water | Batch 1 concentration (mg/L; 1dd/25Gml) | Batch 2 concentration (mg/L; 1dd/25ml) | Batch 3 concentration (mg/L; 1dd/2.5ml |
|---|---|---|---|---|---|---|
| Mn | 0.025 | MnCl₂, 4H2O | 634 g/L | 0.22 | 2.2 | 22 |
| F | 5 | NaF | 50g/L | 8 | 80 | 800 |
| I | 0.080 | KI | 1.48kg/L | 0.4 | 4 | 40 |
| Mo | 0.014 | Na₂MoO₄, 2H2O | < 840g/L | 0.08 | 0.8 | 8 |
| Cr | 0.014 | CrCl₃, 6H2O | 585g/L | 0.04 | 0.4 | 4 |
| Fe | 0.350 | FeCl₃, 6H2O | 920 g/L | 4 | 40 | 400 |
| Se | 0.035 | Na, Selen ate (Na₂Se O₄) | > 100g/L | 0.28 | 2.8 | 28 |

In the context of the medical product of the invention, the volume of the solution comprising a selenate salt, selenomethionine and/or selenocysteine can be in the range of 1 - 1000 ml, 1.5 - 980, 2 - 960, 2.5 - 940, 3 - 920, 3.5 - 900, 4 - 880, 4.5 - 860, 5 - 840, 6 - 820, 7 - 800, 8 - 780, 9 - 760, 10 - 740, 12 - 720, 14 - 700, 16 - 680, 18 - 660, 20 - 640, 22 - 620, 24 - 600, 26 - 580, 28 - 560, 30 - 540, 32 - 520, 34 - 500, 36 - 480, 34 - 460, 36 - 440, 38 - 420, 40 - 400, 45 - 490, 50 - 480, 55 - 470, 60 - 460, 65 - 450, 70 - 440, 85 - 430, 90 - 420, 95 - 410, 100 - 400, 110 - 390, 120 - 380, 130 - 370, 140 - 360, 150 - 350, 160 - 340, 170 - 330, 180 - 320, 190 - 310, 200 - 300, 210 - 290, 220 - 280, 230 - 270, 240 - 260 or 250 ml. The indicated ranges include the noted end-values. Ranges comprising any combination of disclosed end-values are considered as embodiments of the invention.

In embodiments of the invention, the solution is contained in one chamber of a multi chamber container having at least two, at least three, at least four, at least five or at least six chambers.

In embodiments, the container comprises at least a first chamber containing a carbohydrate formulation, a second chamber containing an amino acid formulation, a third chamber containing a lipid formulation, optionally comprises electrolytes and/or vitamins in at least the first, second and/or third chamber, and wherein at least one chamber comprises a selenate salt, selenomethionine or selenocysteine.

In embodiments, the container comprises at least a first chamber containing a carbohydrate formulation, a second chamber containing an amino acid formulation, a third chamber containing a lipid formulation, comprises electrolytes and/or vitamins in at least the first, second and/or third chamber, and wherein at least one chamber comprises a selenate salt, selenomethionine and/or selenocysteine.

In embodiments, the container comprises at least a first chamber containing a carbohydrate formulation, a second chamber containing an amino acid formulation, a third chamber containing a lipid formulation, comprises electrolytes and/or vitamins in a fourth and/or fifth chamber and a selenate salt, selenomethionine and/or selenocysteine in a further chamber, optionally together with other trace elements.

In embodiments, the container comprises at least a first chamber containing a carbohydrate formulation, a second chamber containing an amino acid formulation, a third chamber containing a lipid formulation, and the solution comprising a selenate salt, selenomethionine and/or selenocysteine is present in a fourth chamber.

In embodiments of the invention, where the solution is contained in one chamber of a multi chamber container having at least two, at least three, at least four, at least five or at least six chambers, wherein the solution comprises a selenate salt, preferably sodium selenate or potassium selenate. In embodiments of the invention, the container is made from, a flexible material.

In certain embodiments comprising the container with at least a first chamber containing a carbohydrate formulation, a second chamber containing an amino acid formulation, a third chamber containing a lipid formulation, the solution comprises a selenate salt, preferably sodium selenate or potassium selenate. In embodiments of the invention, the container is made from a flexible material.

In embodiments of the invention, the solution is prepared by dissolving a selenate salt, selenomethionine and/or selenocysteine in a liquid medium. In preferred embodiments, the liquid medium is water, preferably ultrapure water (UPW; also called deionized (DI) water) or water for injection. UPW water has organic particles and dissolved gases removed by techniques known in the art. Water for injection is water of extra high quality without significant contamination.

The invention further relates to a method of preparing a medical product for preventing or correcting selenium deficiency in a patient according to any one of the preceding claims, wherein the solution is prepared by the steps comprising
a. dissolving a selenate salt, selenomethionine and/or selenocysteine in a liquid medium, preferably water for injection, to produce a solution, with a concentration of selenium between 0.28 and 28 mg/L,
b. optionally adjusting the solution to an acidic pH, preferably by providing an organic acid, such as malic acid, and optionally adding at least one additional trace element, preferably selected from the group comprising zinc, iron, copper, manganese, chromium, iodine, fluoride and molybdenum, and
c. sterilizing the solution, preferably by heat sterilization.

Sterilizing of the solution may occur prior or after filling the solution into a flexible container of the invention.

The optional method step of adjusting the pH of the solution to an acidic pH may be achieved by dissolving an acid, such as an organic acid selected from the group comprising malic acid, tartaric acid, citric acid, maleic acid and fumaric acid.

Furthermore, in embodiments of the method of the invention at least one additional trace element, preferably selected from the group consisting of zinc, iron, copper, manganese, chromium, iodine, fluoride and molybdenum is added to the solution.

The invention further relates to a sterile or sterilized solution for parenteral administration comprising at least one compound selected from the group comprising a selenate salt, selenomethionine and/or selenocysteine for use in preventing or correcting selenium deficiency in a patient.

Furthermore, the invention relates to a method of preventing or correcting selenium deficiency in a patient, the method comprising administration of a solution comprising at least one compound selected from the group consisting of a selenate salt, selenomethionine and/or selenocysteine to said patient.

In embodiments of the method of preventing or correcting selenium deficiency in a patient according to the present invention, administration of the solution is for maintaining plasma selenium levels and prevention of depletion of endogenous stores in a patient receiving total parenteral nutrition.

In the context of the method of preventing or correcting selenium deficiency in a patient, the patients according to one embodiment are adult patients. According to another embodiment, the patients are pediatric patients.

As used herein, the term "adult" as used herein refers to persons of 20 years of age and older. The term "pediatric" refers to neonates, including premature (pre-term), full term, and post-mature neonates of up to one month of age; infants of between one month and one year of age; children of between one and up to 12 years of age, and adolescents of between 13 and up to 19 years of age.

All features disclosed in the context of the medical product for preventing or correcting selenium deficiency in a patient of the invention also relate to the method of preparing such a medical product and are herewith disclosed also in the context of the method of preparing the medical product and the other way around. The same holds true for the sterile or sterilized solution of the invention and the method of preventing or correcting selenium deficiency in a patient according to the present invention.

### DETAILED DESCRIPTION OF THE INVENTION

All cited documents of the patent and non-patent literature are hereby incorporated by reference in their entirety.

The present invention is directed to a medical product for preventing or correcting selenium deficiency in a patient comprising a solution provided in a flexible container comprising at least one compound selected from the group comprising or consisting of a selenate salt, selenomethionine and/or selenocysteine.

As disclosed herein, the term medical product relates to any product intended to be used for medical purposes, preferably for clinical nutrition. The medical product of the invention is intended for the medical purpose of preventing or correcting selenium deficiency in a patient.

Selenium deficiency refers to a condition of a patient who does not have enough selenium in his body. This can cause several health problems. The patient group of the invention comprises all patients or patient groups that are at risk of developing a selenium deficiency or have already developed a selenium deficiency. In particular, the medical product of the invention is intended to correct or treat a selenium deficiency that has already occurred in a patient. Furthermore, the invention may be used to prevent the occurrence of selenium deficiency in patients at risk of or suspected of developing a selenium deficiency, for example due to severely compromised intestinal function, total or partial parenteral nutrition, gastrointestinal bypass surgery, or advanced age. In the present context of the invention, prevention is considered to encompass both absolute prevention, i.e. stopping of a disease developing at all, and/or a preventative measure that lowers the risk or likelihood of the patient developing the unwanted medical conditions or slows or delays the development, first occurrence and/or progression of the disease.

Selenium is a chemical element with the symbol Se and atomic number 34. It is a nonmetal (more rarely considered a metalloid) with properties that are intermediate between the elements above and below in the periodic table, sulfur and tellurium, and also has similarities to arsenic. Selenium itself exists in a variety of chemical forms including selenite and selenate as well as elemental selenium and it is often found associated with sulfur-containing compounds. Selenium is a component of the amino acids selenocysteine and selenomethionine. Very small quantities of selenium are required to maintain proper health in both animals and humans and this selenium must be obtained through dietary sources. There are around 25 seleno-proteins in humans and many of these are enzymes that act to protect the body against oxidative damage. Without selenium, the function of the selenium-requiring proteins can be compromised which results in the signs and symptoms of deficiency. Since the ageing process, as well as certain diseases, including cancer and cardiovascular disease, are associated with an increase in oxidative damage, maintaining adequate selenium intakes may provide some protection against these processes.

In humans, selenium is a trace element nutrient that functions as cofactor for glutathione peroxidases and certain forms of thioredoxin reductase. Selenium-containing proteins are produced from inorganic selenium via the intermediacy of selenophosphate (PSeO₃³⁻).

Selenium deficiency can occur in patients with severely compromised intestinal function, such as those undergoing total parenteral nutrition, patients undergoing CRRT, cancer patients, preterm infants, patients staying in an ICU for a prolonged time, those who have had gastrointestinal bypass surgery, and in those of advanced age (over 90). These patients are at risk of developing selenium deficiency. Also, people dependent on food grown from selenium-deficient soil are at risk. Increased risk for developing various diseases has also been noted, even when certain individuals lack optimal amounts of selenium, but not enough to be classified as deficient.

The medical product of the invention can be used in the treatment of patients having, developing or being at risk of developing selenium deficiency. The treatment can aim to prevent or correct a selenium deficiency in this patient group.

Selenium deficiency, which may be defined by low (<60% of normal) selenoenzyme activity levels in brain and endocrine tissues, occurs when a low selenium level is linked with an additional stress, such as high exposures to mercury or increased oxidant stress from vitamin E deficiency. Selenium deficiency as used herein can also occur in healthy well-nourished individuals. Selenium deficiency in combination with Coxsackievirus infection can lead to Keshan disease, which is potentially fatal and represents a specific form of selenium deficiency in the sense of the invention. Selenium deficiency also contributes (along with iodine deficiency) to Kashin-Beck disease, which is another selenium deficiency in the sense of the invention. The primary symptom of Keshan disease is myocardial necrosis, leading to weakening of the heart. Kashin-Beck disease results in atrophy, degeneration and necrosis of cartilage tissue. Keshan disease also makes the body more susceptible to illness caused by other nutritional, biochemical, or infectious diseases. Selenium is also necessary for the conversion of the thyroid hormone thyroxine (T4) into its more active counterpart triiodothyronine (T3). Therefore, selenium deficiency can cause symptoms of hypothyroidism, including extreme fatigue, mental slowing, goiter, cretinism, and recurrent miscarriage.

The U.S. Institute of Medicine (IOM) updated Estimated Average Requirements (EARs) and Recommended Dietary Allowances (RDAs) for selenium in 2000. If there is not sufficient information to establish EARs and RDAs, an estimate designated Adequate Intake (AI) is used instead. The current EAR for selenium for people ages 14 and up is 45 µg/day. The RDA is 55 µg/day. RDAs are higher than EARs so as to identify amounts that will cover people with higher than average requirements. RDA for pregnancy is 60 µg/day. RDA for lactation is 70 µg/day. For children ages 1-13 years the RDA increases with age from 20 to 40 µg/day. As for safety, the IOM sets Tolerable upper intake levels (ULs) for vitamins and minerals when evidence is sufficient. In the case of selenium the UL is 400 µg/day. Collectively the EARs, RDAs, AIs and ULs are referred to as Dietary Reference Intakes (DRIs) [Institute of Medicine (2000). "Selenium". Dietary Reference Intakes for Vitamin C, Vitamin E, Selenium, and Carotenoids. Washington, DC: The National Academies Press. pp. 284-324]. The European Food Safety Authority (EFSA) refers to the collective set of information as Dietary Reference Values, with Population Reference Intake (PRI) instead of RDA, and Average Requirement instead of EAR. AI and UL defined the same as in United States. For women and men ages 15 and older the AI is set at 70 µg/day. AI for pregnancy is 70 µg/day, for lactation 85 µg/day. For children ages 1-14 years the AIs increase with age from 15 to 55 µg/day. These AIs are higher than the U.S. RDAs [EFSA, Overview on Dietary Reference Values for the EU population as derived by the EFSA Panel on Dietetic Products, Nutrition and Allergies (NDA), September 2017]. The European Food Safety Authority (EFSA) reviewed the same safety question and set its UL at 300 µg/day, which is lower than the U.S. value [EFSA, "Tolerable Upper Intake Levels For Vitamins And Minerals", February 2006].

In the context of the invention, the medical product comprises a flexible container or bag comprising at least one compound selected from the group consisting of a selenate salt, selenomethionine and/or selenocysteine.

The selenate ion is SeO₄²⁻. Selenates are analogous to sulfates and have similar chemistry. They are highly soluble in aqueous solutions at ambient temperatures. In strongly acidic conditions, the hydrogen selenate ion, HSeO₄⁻, is formed. It corresponds to the selenic acid, H₂SeO₄, which is a strong acid. Unlike sulfate, selenate is a somewhat good oxidizer; it can be reduced to selenite or selenium. Selenate is a component of various salts, such as for example sodium selenate, potassium selenate, barium selenate and ammonium selenate.

Sodium selenate is the inorganic compound with the formula Na₂SeO₄, not to be confused with sodium selenite. It exists as the anhydrous salt, the heptahydrate, and the decahydrate. Sodium selenate can be produced by oxidation of selenium, first with nitric acid, producing selenous acid. The selenous acid is neutralized to form sodium selenite. The sodium selenite is oxidized in a basic medium hydrogen peroxide to form a selenate, which is then spraydried.

Potassium selenate, K₂SeO₄, is an odorless, white solid that forms as the potassium salt of selenic acid. Potassium selenate can be produced by the reaction of selenium trioxide and potassium hydroxide. Alternatively, it can be made by reacting selenous acid with potassium hydroxide, and then oxidizing the resulting potassium selenite with bromine.

Selenomethionine (SeMet) is a naturally occurring amino acid. The L-selenomethionine enantiomer is the main form of selenium found in Brazil nuts, cereal grains, soybeans, and grassland legumes, while Se-methylselenocysteine, or its γ-glutamyl derivative, is the major form of selenium found in Astragalus, Allium, and Brassica species. In vivo, selenomethionine is randomly incorporated instead of methionine. Selenomethionine is readily oxidized. Selenomethionine has antioxidant activity that arises from its ability to deplete reactive oxygen species. Selenium and methionine also play separate roles in the formation and recycling of glutathione, a key endogenous antioxidant in many organisms, including humans.

Selenocysteine (symbol Sec or U or Se-Cys) is considered the 21st proteinogenic amino acid and exists naturally in all three domains of life, but not in every lineage, as a building block of selenoproteins. Selenocysteine is a cysteine analogue with a selenium-containing selenol group in place of the sulfur-containing thiol group. Selenocysteine is present in several enzymes, such as glutathione peroxidases, tetraiodothyronine 5' deiodinases, thioredoxin reductases, formate dehydrogenases, glycine reductases, selenophosphate synthetase 2, methionine-R-sulfoxide reductase B1 (SEPX1), and some hydrogenases.

Sodium selenite is the inorganic compound with the formula Na₂SeO₃. This salt is a colorless solid. The pentahydrate Na₂SeO₃(H₂O)₅ is the most common water-soluble selenium compound. Selenous acid (or selenious acid) is the chemical compound with the formula H₂SeO₃. Structurally, it is more accurately described by H₂SeO₃. It is the principal oxoacid of selenium; the other being selenic acid. Selenium dioxide is the chemical compound with the formula SeO₂ and is a colorless solid. It is one of the most frequently encountered compounds of selenium.

In embodiments of the invention, the solution of the medical product is a sterilized solution. In the context of the invention, the term "sterilized" relates to a solution that has undergone a process of sterilization. Sterilization refers to any process that eliminates, removes, kills, or deactivates all forms of life (in particular referring to microorganisms such as fungi, bacteria, viruses, spores, unicellular eukaryotic organisms such as Plasmodium, etc.) and other biological agents like prions present in a specific surface, object or fluid, for example food or biological culture media. Sterilization can be achieved through various means, including heat, chemicals, irradiation, high pressure, and filtration. Sterilization is distinct from disinfection, sanitization, and pasteurization, in that those methods reduce rather than eliminate all forms of life and biological agents present. After sterilization, an object is referred to as being sterile or aseptic.

According to one embodiment of the invention, sterilization is done by heat. According to another embodiment of the invention, methods encompass sterilization with moist heat. The term "moist heat" as used herein includes the use of saturated steam with or without pressure, steam air and hot water cascade or water spray sterilization. According to one embodiment of the invention, sterilization with moist heat is preferable. Generally, said sterilization with moist heat can be used for drug products, medical devices, plastic bags and other single-use equipment, glass containers, surgical dressings and more.

Sterilization can also be achieved via dry heating. Much higher temperatures (180-200 °C) are required for this method. Dry heating is commonly used to sterilize glassware, metal and other surfaces.

Exposure to radiation is another sterilization method used throughout the industry. Gamma radiation is the most common, though other options include infrared and ultraviolet radiation and high-velocity electrons. Radiation is typically used for the sterilization of single-use components/systems, but it can be used for packaged drug products.

Treatment with gases is also a sterilization alternative. Such gases include ethylene oxide, formaldehyde, glutaraldehyde, propylene oxide, hydrogen peroxide and chlorine dioxide. This method is more commonly used to sterilize clean-room suites. Sterilization via filtration is the only option if the other processes are not suitable for the specific product or component. In filtration, the final drug product solution is passed through a filter that has been produced under aseptic manufacturing conditions and designed with appropriate pore sizes/surface chemistries that remove bacteria via size exclusion, entrapment, electrostatic attraction and other modalities.

In embodiments of the invention, the solution comprises an acid, preferably an organic acid selected from the group comprising malic acid, tartaric acid, citric acid, maleic acid, fumaric acid, more preferably malic acid.

As used herein, a nutrient is a substance used by an organism, such as a human, to survive, grow, and reproduce. Nutrients can be incorporated into cells for metabolic purposes or excreted by cells to create non-cellular structures, such as hair, scales, feathers, or exoskeletons. Some nutrients can be metabolically converted to smaller molecules in the process of releasing energy, such as for carbohydrates, lipids, proteins/amino acids, and fermentation products (ethanol or vinegar), leading to end-products of water and carbon dioxide. All organisms require water. Essential nutrients for animals and humans are the energy sources, some of the amino acids that are combined to create proteins, a subset of fatty acids, vitamins and certain minerals/trace elements.

A classification used primarily to describe nutrient needs of animals divides nutrients into macronutrients and micronutrients. Consumed in relatively large amounts, macronutrients (carbohydrates, lipids/fats, proteins/amino acids, water) are used primarily to generate energy or to incorporate into tissues for growth and repair. Micronutrients are needed in smaller amounts; they have subtle biochemical and physiological roles in cellular processes, like vascular functions or nerve conduction. Inadequate amounts of essential nutrients, or diseases that interfere with absorption, result in a deficiency state that compromises growth, survival and reproduction.

Macronutrients comprise carbohydrates, proteins, lipids, and water. Macronutrients are defined as a class of chemical compounds which humans consume in the largest quantities and which provide humans with the bulk of energy. While water does make up a large proportion of the total mass ingested as part of a normal diet, it does not provide any nutritional value. Carbohydrates comprise glucose, sucrose, ribose, amylose (a major component of starch), amylopectin, maltose, galactose, fructose, lactose. Proteins are composed of amino acids comprising standard amino acids alanine, arginine, aspartic acid (aspartate), asparagine, cysteine, glutamic acid (glutamate), glutamine, glycine, histidine, isoleucine (branched chain amino acid), leucine (branched chain amino acid), lysine, methionine, phenylalanine, proline, serine, threonine, tryptophan, tyrosine, valine (branched chain amino acid). Lipids (or fats) comprise saturated fats such as butyric acid (C4), caproic acid (C6), caprylic acid (C8), capric acid (C10), lauric acid (C12), myristic acid (C14), pentadecanoic acid (C15), palmitic acid (C16), margaric acid (C17), stearic acid (C18), arachidic acid (C20), behenic acid (C22), lignoceric acid (C24), cerotic acid (C26); monounsaturated fats such as myristol, pentadecanoic, palmitoyl, heptadecenoic, oleic acid, eicosen, erucic acid, nervonic acid; polyunsaturated fats such as linoleic acid (LA) - an essential fatty acid, α-Linolenic acid (ALA) - an essential fatty acid, stearidonic acid (SDA), arachidonic acid (ETA), timnodonic acid (EPA), clupanodonic acid (DPA), cervonic acid (DHA); essential fatty acids, such as α-Linolenic acid ALA (18:3) Omega-3 fatty acid and Linoleic acid LA (18:2) Omega-6 fatty acid, being the starting point for other important omega-acids (e.g. DHA, EPA).

Micronutrients are essential elements required by human in small quantities throughout life to orchestrate a range of physiological functions to maintain health, in particular vitamins and dietary minerals/trace elements. Trace elements/trace minerals/dietary minerals include Boron, Cobalt (as a component of vitamin B12), Fluoride, Chromium, Copper, Iodine, Iron, Manganese, Molybdenum, Selenium and Zinc.

In the context of the medical product of the invention, trace elements may be provided as chloride, potassium or sodium salts, such as zinc chloride, iron chloride, copper chloride, sodium selenate, manganese chloride, sodium fluoride, potassium iodide, chromium chloride, sodium molybdate.

Vitamins comprise Vitamin B complex, Vitamin B1 (thiamin), Vitamin B2 (riboflavin), Vitamin B3 (niacin), Vitamin B5 (pantothenic acid), Vitamin B6 group (Pyridoxine, Pyridoxal-5-Phosphate, Pyridoxamine), Vitamin B7 (biotin), Vitamin B9 (folate), Vitamin B12 (cobalamin), Choline, Vitamin A (e.g. retinol (see also - provitamin A carotenoids)), Vitamin C (Ascorbic acid), Vitamin D (vitamin D2 (Ergocalciferol), vitamin D3 (Cholecalciferol)), Vitamin E (tocopherols and tocotrienols), Vitamin K (Vitamin K1 (phylloquinone), Vitamin K2 (menaquinone)).

Electrolytes, such as sodium, potassium, chloride, calcium, magnesium, phosphate and bicarbonate may also be classified as nutrients.

The medical product of the invention comprises a solution provided in a flexible container comprising at least one compound selected from the group consisting of a selenate salt, selenomethionine and/or selenocysteine. The medical product may comprise further solutions comprising nutrients, such as macronutrients and micronutrients. The solutions of the medical product may be reconstituted before administration to a patient. Administration of the one or more solutions of the product, potentially after reconstitution of the one or more solutions, can occur through various routes of administration, such as parenterally, orally or enterally.

As used herein, "reconstituted solution" refers to a solution for parenteral administration, which is generated by admixing the content of the chambers of a multi-chamber container before use.

Parenteral administration is preferred in the context of the invention. Parenteral nutrition (PN) is the feeding of specialist nutritional products to a person intravenously, bypassing the usual process of eating and digestion. It is called total parenteral nutrition (TPN) or total nutrient admixture (TNA) when no significant nutrition is obtained by other routes, and partial parenteral nutrition (PPN) when nutrition is also partially enteric. It may be called peripheral parenteral nutrition (PPN) when administered through vein access in a limb rather than through a central vein as central venous nutrition (CVN). Enteral food administration is via the human gastrointestinal tract and contrasts with parenteral administration.

In the context of the medical product of the invention, the solution is provided in a flexible container. In embodiments of the invention, the solution is contained in one chamber of a multi chamber container having at least two, at least three, at least four, at least five or at least six chambers.

As used herein, the term "flexible container" refers to a container or bag made of a flexible material, such as bags made from plastic films. The term does not encompass polymeric rigid or semirigid containers.

Flexible containers or bags of the invention can be made of materials comprising, without limitation, polyvinyl chloride (PVC), polypropylene (PP), polyethylene (PE), ethylene vinyl alcohol (EVOH), ethylene-vinyl acetate (EVA) and all possible copolymers, essentially any synthetic material suitable for containing the components to be administered.

For example, oxygen impermeable flexible containers are made of gas barrier films that block oxygen migration to the outside of the container. Different technologies have been developed in order to provide oxygen barrier to transparent films, such as PE films or polyethylene terephthalate films. The main technologies are the following: (1) Coating with high barrier materials, generally inorganic oxide layers (e.g. SiOx or Al₂O₃); (2) Multilayer films, wherein an inner layer consists a barrier material such as EVOH, polyamide, aluminum, halogenated polyvinylidene such as PVDC, amorphous nylon or crystalline nylon or combination of both, copolymers of ethylene vinyl alcohol copolymer layer (EVOH), polyolefins, including combinations of two or more of the above layers, and wherein the outer layers consist of structural polymer (e.g. PE, PP or PET).

The disclosure therefore also provides for a flexible container, preferably a multi-chamber container for parenteral or nutritional formulations which may be prepared from any of the before-mentioned flexible films. For example, the container may be in the form of a bag having one or multiple compartments or chambers. The container, such as a bag, may include at least two chambers, but may also contain three, four, five or six or more chambers, and in one preferred embodiment, two or three chambers.

Suitable containers, including soft bags, typically are sterile, non-pyrogenic, single-use, and/or ready-to-use. The multi-chamber containers are particularly useful for holding a parenteral nutrition product for adults, children or neonates and can provide a carbohydrate formulation as disclosed herein in the first chamber, an amino acid formulation as disclosed herein in a second chamber, and a lipid formulation as disclosed herein in a third chamber of the container.

The multi-chamber container may include vertical chambers as disclosed in U.S. Patent Publication No. 2007/0092579. For example, the multi-chamber container may be configured as a bag that includes two, three, four, five or six adjacent chambers or compartments. If desired, frangible barriers or openable seals (e.g., peel seals or frangible seals) are used to separate the chambers of the multi-chamber container. Multi-chamber containers may also comprise three chambers for accommodating a lipid emulsion, a carbohydrate formulation and an amino acid formulation, and further comprise at least one, in certain embodiments two or three smaller chambers which contain, for example, vitamin formulations and/or trace element formulations. In one specific embodiment, the multi-chamber container of the invention has a first chamber containing the lipid emulsion according to the invention, a second chamber containing an amino acid formulation, a third chamber containing a carbohydrate formulation, a fourth chamber containing a vitamin formulation and a fifth chamber containing a trace element formulation.

A multi chamber container or multi chamber bag (MCB) as used in the context of the invention may be designed for the parenteral administration of its reconstituted content after admixing the formulations contained in the respective chambers. Such MCB may have 2, 3, 4, 5, 6 or more chambers. The chambers of said MCB may have the same size or may have different sizes to accommodate various compositions and volumes. The chambers may be designed to contain volumes of from, for ex-ample, 1 to 5 ml, from 5 to 10 ml, from 10 to 50 ml, from 50 to 100 ml, from 100 to 250 ml, from 250 ml to 500 ml, from 500 to 1000 ml, from 1000 to 1500 ml. The MCBs can be designed to have chambers which are located adjacent to each other. The chambers may have various shapes. The chambers can be oriented horizontally and/or vertically to each other. Certain small chambers can be designed to be located within another, larger chamber, wherein, for example, the small chamber which is located within another, larger chamber can be accommodated and fixed into said larger chamber by welding at least one edge of said small chamber in between the weld seam of the surrounding larger chamber.

The openable seals of said multi-chamber containers permit formulations to be separately stored and admixed/reconstituted just prior to administration thereby allowing storage in a single container of formulations which should not be stored as an admixture for an extended period of time. Opening of the seals allows communication between the chambers and mixing of the contents of the respective chambers. The outside seals of the multi-chamber container are strong seals that do not open under the fluid pressure supplied to open the weaker peel seals or frangible seals between the chambers. In some embodiments, the openable seals of the multi-chamber container may be designed to allow for the admixing or reconstitution of only selected chambers of the multi-chamber container, for example, the admixing of the lipid emulsion with the vitamin chamber and the amino acid chamber, if so desired.

The multi-chamber container may be provided with instructions explaining a desired order with which to open the peel seals, so that constituent fluids are mixed in a desired order. The unsealing strengths of the two or more peel seals may be varied to promote the opening of the seals in the desired order. For example, the unsealing strength of the peel seal to be opened first may be 1/3 to 1/2 of the unsealing strength required to open the peel seal to be opened second.

In embodiments, the container comprises at least a first chamber containing a carbohydrate formulation, a second chamber containing an amino acid formulation, a third chamber containing a lipid formulation, optionally comprises electrolytes and/or vitamins in at least the first, second and/or third chamber, and wherein at least one chamber comprises at least one of a selenate salt, selenomethionine or selenocysteine.

As used herein, amino acid formulations include a sterile, aqueous solution of one or more amino acids and one or more electrolytes. Typically, amino acid formulations include about 2 grams to about 10 grams of amino acids per 100 mL of amino acid formulation, such as about 3 grams to about 9 grams and/or about 5 grams to about 7 grams per 100 mL of amino acid formulation. Typical amino acids which are included into amino acid formulations are, for example, isoleucine, leucine, valine, lysine, methionine, phenylalanine, threonine, tryptophan, arginine, histidine, alanine, aspartic acid, cysteine, glutamic acid, glycine, proline, serine, tyrosine, ornithine, and taurine. Further, the content of tyrosine can be increased by adding, for example, a glycyl-tyrosine dipeptide or acetyl-tyrosine (Ac-Tyr). Typically, however, the glycyl-tyrosine dipeptide has improved pharmacokinetics compared to Ac-Tyr, which is more rapidly eliminated by the kidney, resulting in diminished release of tyrosine in the blood.

The amino acid formulation may further include electrolytes. As used herein, electrolytes include sodium, potassium, calcium, magnesium, and/or phosphate ions. For example, the amino acid formulation can include from about 0.1 mmol to about 10 mmol of sodium (e.g., about 3.75 mmol to about 10 mmol of sodium), from about 0.1 mmol to about 10 mmol of potassium (e.g., about 3.75 mmol to about 6.90 mmol of potassium), from about 0.05 mmol to about 1.0 mmol of magnesium (e.g., about 0.05 mmol to about 0.11 mmol and/or about 0.38 mmol to about 0.65 mmol of magnesium), from about 0.1 mmol to about 10 mmol of calcium (e.g., about 1.13 mmol to about 5.10 mmol of calcium), from about 0.1 mmol to about 10 mmol of phosphate (e.g., about 0.94 mmol to about 5.10 mmol of phosphate) and not more than 10 mmol of chloride (e.g., not more than 5.6 mmol of chloride) per 100 mL of amino acid formulation. When calcium and phosphorus are present together in the same heat-sterilized solution, insoluble calcium phosphate precipitation can occur. Using an organic salt of phosphorus such as sodium glycerophosphate 5·H₂O or calcium glycerophosphate, calcium and phosphate amounts may be increased without solubility issues and without providing excess sodium or chloride. In the amino acid formulation, sodium may be provided in the form of sodium chloride, calcium may be provided in the form of calcium chloride 2·H₂O or calcium gluconate, magnesium may be provided in the form of magnesium acetate 4·H₂O or magnesium chloride, and potassium may be provided in the form of potassium acetate.

Carbohydrate formulations provide a supply of calories, typically in the form of glucose. In particular, the carbohydrate formulation provides an amount of carbohydrate sufficient to avoid adverse effects such as hyperglycemia that has been observed in patients receiving parenteral nutrition. Typically, the carbohydrate formulation includes about 20 to 50 grams of glucose per 100 mL of carbohydrate formulation.

Lipid formulations such as mentioned in the context of the present invention are an emulsion of an oil phase, a water phase, and an emulsifier that makes the two phases miscible. In case of lipid emulsions, which are to be used as an injectable emulsion for parenteral nutrition, the emulsion must be an oil-in-water (o/w) emulsion. This means that the oil must reside in the internal (or dispersed) phase, while water is the external (or continuous) phase, as the emulsion must be miscible with blood. Lipid emulsion as disclosed herein must therefore also be substantially free of any suspended solids. Of course, the lipid emulsions may contain further components, including, but not limited to, antioxidants, pH modifiers, isotonic agents, vitamins, trace elements and various combinations thereof. An overview over lipid emulsions, their composition and use is provided, for example, in Driscoll, Journal of Parenteral and Enteral Nutrition 2017, 41, 125-134. Further information on the use of lipid emulsions in parenteral nutrition of intensive care patients is provided, for example, in Calder et al, Intensive Care Medicine, 2010, 36(5), 735-749.

The oil phase of the lipid emulsion may include unsaturated and polyunsaturated fatty acids, such as long-chain polyunsaturated fatty acids, which may be present as the free acid, as an ionized or salt form of the free acid, and/or in ester form. Suitable esters of the polyunsaturated fatty acids/long-chain polyunsaturated fatty acids include, but are not limited to, alkyl esters (e.g., methyl esters, ethyl esters, propyl esters, or combinations thereof) and triglyceride esters. In some cases, the long-chain polyunsaturated fatty acid has a structure R(C=O)OR', wherein R is an alkenyl group having at least 17 carbon atoms, at least 19 carbon atoms, at least 21 carbon atoms, or at least 23 carbon atoms, and R' is absent, H, a counter ion, an alkyl group (e.g., methyl, ethyl, or propyl), or a glyceryl group (e.g., R(C=O)OR' is a monoglyceride, a diglyceride, or a triglyceride). Polyunsaturated fatty acids for use in the lipid formulations disclosed herein include, but are not limited to, linoleic acid (LA), arachidonic acid (ARA), α-linolenic acid (ALA), eicosapentaenoic acid (EPA), docosahexaenoic acid (DHA), stearidonic acid (SDA), γ-linolenic acid (GLA), dihomo-γ-linolenic acid (DPA), and docosapentaenoic acid (DPA), particularly, DHA, ARA, and EPA, each of which may be present in free acid form, ionized or salt form, alkyl ester form, and/or triglyceride form. In some cases, the polyunsaturated fatty acids and/or long-chain fatty acids are present in triglyceride form.

Typically, the lipid formulation includes about 5% to about 35% by weight of an oil phase based on the total weight of the lipid emulsion. For example, the oil phase of the lipid emulsion is present in an amount of about 8% to 12%, of about 10% to about 20%, of about 10% to about 15%, of about 15% to about 20%, of about 12% to about 17%, of about 18% to 22% and/or about 20% by weight based on the total weight of the lipid formulation. The oil phase typically and preferably contains, in various amounts depending on the source of the oil, omega-3 fatty acids. The three types of omega-3 fatty acids involved in human metabolism are eicosapentaenoic acid (EPA) and docosahexaenoic acid (DHA), both of which are usually found in marine fish oils and α-linolenic acid (ALA), commonly found in plant oils.

The lipid emulsions referred to herein may further include additional components, such as surfactants (also referred to as emulsifiers), co-surfactants, isotonic agents, pH adjusters, and antioxidants. Generally, surfactants are added to stabilize emulsions by reducing the interfacial tension between the oil phase and the aqueous phase. Surfactants typically include a hydrophobic part and a hydrophilic part, and the amount of surfactant/emulsifier included in the formulations is determined based on the amount that is needed to achieve a desired level of stabilization of the emulsion. Typically, the amount of surfactant in the lipid formulation is about 0.01 % to about 3% by weight based on the total weight of the lipid formulation, for example, about 0.01 % to about 2.5%, about 0.01 % to about 2.3%, about 0.02 % to about 2.2%, about 0.02 % to about 2.1%, about 0.02 % to about 2%, about 0.05% to about 1.8%, about 0.1% to about 1.6%, about 0.5% to about 1.5%, about 0.8% to about 1.4%, about 0.9% to about 1.3%, about 1% to about 1.2%, and/or about 1.2% by weight. Suitable surfactants and co-surfactants include surfactants that are approved for parenteral use, and include, but are not limited to, phospholipids (e.g., egg phosphatide and soy lecithin), oleate salts, and combinations thereof. Krill oil can also be used as an emulsifier in the lipid emulsion, wherein the lipid emulsion comprises about 0.5 to 2.2 wt % krill oil based on the total weight of the emulsion, and wherein the emulsion is free of egg yolk lecithin (US 2018/0000732 A1). Another exemplary surfactant is lecithin, including both natural and synthetic lecithin, such as lecithins derived from egg, corn or soybean or mixtures there-of. In some cases, lecithin is included in an amount of about 1.2% based on the total weight of the lipid formulation.

In some cases, the lipid emulsion formulation includes a co-surfactant. Typically, the amount of co-surfactant in the lipid formulation is less than the amount of surfactant, and typically the amount of co-surfactant in the formulation is about 0.001% to about 0.6% by weight based on the total weight of the lipid formulation, for example, about 0.001% to about 0.55%, about 0.001% to about 0.525%, about 0.001% to about 0.5%, about 0.005% to about 0.5%, about 0.01% to about 0.4%, about 0.02% to about 0.3%, about 0.03% to about 0.2%, about 0.04% to about 0.1%, and/or about 0.05% to about 0.08%. An exemplary co-surfactant is oleate, such as sodium oleate. In some cases, the lipid formulation includes lecithin and oleate as surfactant and co-surfactant, for example, an in amount of 1.2% lecithin and 0.03% oleate. In some cases, sodium oleate is included in an amount of about 0.03% by weight based on the total weight of the lipid formulation.

Isotonic agents can be added to the lipid emulsions to adjust the osmolarity of the lipid emulsion to a desired level, such as a physiologically acceptable level. Suitable isotonic agents include, but are not limited to, glycerol. Typically, the lipid emulsion formulation has an osmolarity of about 180 to about 300 milliosmols/liter, such as about 190 to about 280 milliosmols/liter, and/or about 200 to about 250 milliosmols/liter. In some cases, the lipid emulsion includes an isotonic agent in an amount of about 1% to about 10% by weight based on the total weight of the lipid formulation, such as about 1% to about 5%, about 1% to about 4%, and/or about 2% to about 3%. In some cases, the lipid emulsion formulation includes about 2% to about 3% by weight of glycerol.

pH modifiers can be added to the lipid emulsions to adjust the pH to a desired level, such as a physiologically acceptable pH for parenteral use. Suitable pH modifiers include but are not limited to sodium hydroxide and hydrochloric acid. Typically, the lipid emulsion formulation has a pH of about 6 to about 9, such as about 6.1 to about 8.9, about 6.2 to about 8.8, about 6.3 to about 8.7, about 6.4 to about 8.6, about 6.5 to about 8.5, about 6.6 to about 8.4, about 6.7 to about 8.3, about 6.8 to about 8.2, about 6.9 to about 8.1, about 7 to about 8, about 7.1 to about 7.9, about 7.2 to about 7.8, about 7.3 to about 7.7, about 7.4 to about 7.6, about 7, about 7.5, and/or about 8.

The lipid formulations may further include antioxidants. Suitable antioxidants may be pharmaceutically acceptable antioxidants and include, but are not limited to, tocopherols (e.g., gamma tocopherol, delta tocopherol, alpha tocopherol), ascorbyl palmitate, or combinations thereof. In some cases, the lipid emulsion formulation includes an antioxidant in an amount of about 0 to about 200 mg/L, for example, about 10 to about 200 mg/L, about 40 to about 150 mg/L, about 50 to about 120 mg/L, about 75 to about 100 mg/L antioxidant(s), such as vitamin E.

The aqueous (or water) phase of all intravenous lipid emulsions must conform to the pharmacopeial requirements that make it suitable for injection, that is, the water must be sterile water for injection.

The lipid emulsions can be prepared according to generally known processes (see, for example, Hippalgaonkar et al, AAPS PharmSciTech 2010, 11(4), 1526-1540 or WO 2019/197198 A1). Generally, water soluble and oil-soluble ingredients are dissolved in the aqueous phase and oil phase, respectively.

If not defined otherwise herein, all terms used in the context of the present invention are to be interpreted according to the understanding of a person skilled in the art.

### FIGURES

The invention is further described by the following figures. These are not intended to limit the scope of the invention but represent preferred embodiments of aspects of the invention provided for greater illustration of the invention described herein.

### Description of the figures:

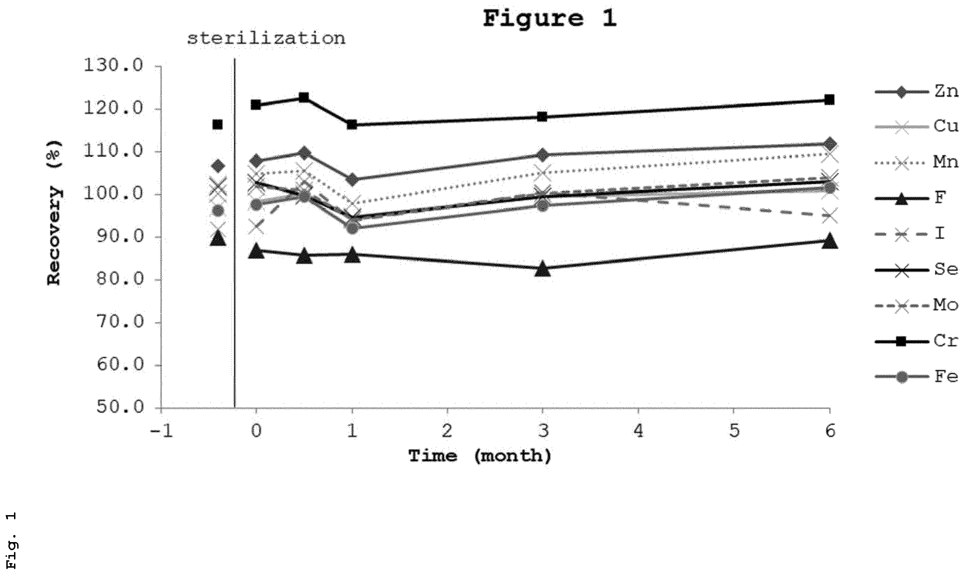

Figures 1-3: The recovery of the initial concentration of the different trace elements in percent (%) is indicated on the Y-axis of the Figures. The analyzed timepoints are is indicated on the X-axis. The timepoint -0.5 corresponds to the results of TE dosage on unsterilized sample at T0. Timepoint 0 corresponds to the results of TE dosage right after sterilization. Timepoint 0.5 correspond to the results of TE dosage after an accelerated stability stress cycle (i.e. three repetition of alternatively 5 °C/50 °C, 48 h/48 h). Timepoints 1, 3 and 6 correspond to the results of TE dosage after 1 month, 3 months and 6 months storage at 40 °C/25% RH.
Figure 1 refers to a solution containing selenium as sodium selenate.
Figure 2 refers to a solution containing selenium as selenomethionine.
Figure 3 refers to a solution containing selenium as selenite.

### EXAMPLES

The invention is further described by the following examples. These are not intended to limit the scope of the invention but represent preferred embodiments of aspects of the invention provided for greater illustration of the invention described herein.

### Example 1: Lab-scale manufacturing of a selenium containing solution for parenteral nutrition or administration

For preparing a selenium containing solution according to the invention the required amount of organic acid is dissolved in water for injection and the pH of the solution is adjusted to the target pH ±0.5 with NaOH or HCl, as needed. In a next step the selenium as sodium selenate, selenomethionine or selenocysteine is introduced into the solution and stirred or agitated until complete dissolution. Afterwards, the pH is again controlled and, if needed, finally adjusted to the target pH ±0.2 with NaOH or HCl. The solution is then filled into the container successively closed/sealed and can undergo a terminal heat sterilization.

### Example 2: Lab-scale manufacturing of a multi trace element solution for parenteral nutrition or administration containing selenium

For preparing a multi trace element selenium containing solution for parenteral nutrition/administration the required amount of organic acid is dissolved in water for injection and the pH of the solution is adjusted to the target pH ±0.5 with NaOH or HCl, as needed. In a next step the required amount of each trace element (including sodium selenate and/or selenomethionine and/or selenocysteine) weighed in and added to the solution under constant stirring or agitation until complete dissolution. If, for certain trace elements, the required quantity is too low for weighing in the required amount, an intermediate concentrated solution can be previously prepared. A given volume of this intermediate solution will then be added to the final solution to reach the target concentration. Afterwards, the pH is again controlled and, if needed, finally adjusted to the target pH ±0.2 with NaOH or HCl. Samples are filled and then the container is hermetically closed/sealed and can undergo to a terminal heat sterilization.

### Example 3: Stabilization of selenium by using a selenate salt, selenomethionine or selenocysteine.

Selenate was introduced with other trace elements into a separate chamber from macronutrients. A better stability of all the ingredients was achieved at acid pH 1 to 4 preferably between 2.2 and 3.5.

A better stability was achieved if an organic acid was added, for example malic acid, tartaric acid, citric acid, maleic acid or fumaric acid.

Each of sodium selenate, selenomethionine and selenocysteine remained stable when introduced into a specific separated chamber alone or together with other trace elements in solution (after 6 months storage at 40 °C/25% RH).

Another way of introduction would be to include Sodium selenate and/or selenomethionine into the glucose, amino acid or lipid chamber.

### Example 4: Assessment of selenium stability using solutions containing selenate, selenomethionine or selenite

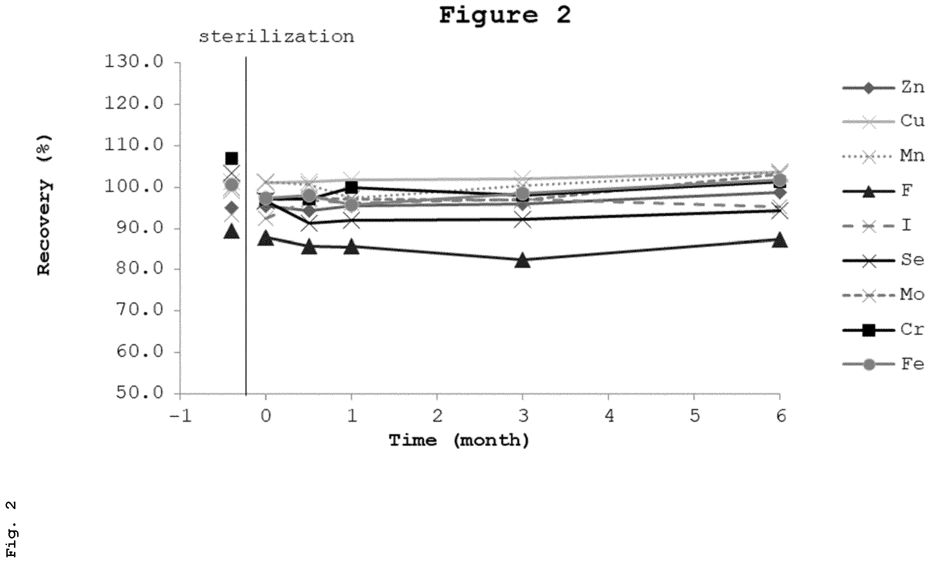

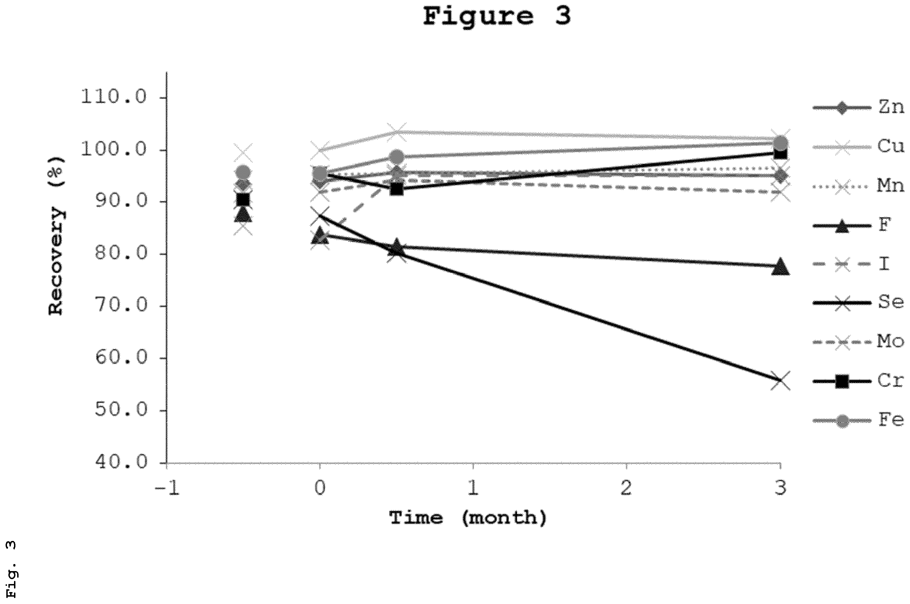

Figures 1-3 illustrate the better stability of selenium when introduced as selenate (Figure 1) or selenomethionine (Figure 2) compared with selenite (Figure 3) after storage for three months. Selenate and selenomethionine remained stable for up to 6 months. For selenite, analysis was stopped after three months due to lack of stability.

For the three tested solutions containing different forms of selenium, the selenium is mixed with other TE (Zn, Cu, Mn, F, I, Mo, Cr, Fe), which were introduced as chloride salts. The solutions were acidified to pH 2.2 using malic acid and underwent terminal heat sterilization prior to storage.

The results as shown in Figures 1-3 demonstrate that the selenium concentration remained stable in solutions comprising selenate or selenomethionine, whereas the selenium concentration decreased significantly in the solution comprising selenium as selenite. Concentrations of all other TE remained about stable under all tested conditions.

## Claims

1. A medical product for preventing or correcting selenium deficiency in a patient comprising a solution provided in a flexible container **characterized in that** the solution comprises at least one of a selenate salt and/or a seleno-amino acid, such as selenomethionine or selenocysteine.

2. The medical product according to the preceding claim, wherein the selenate salt and/or seleno-amino acid, such as selenomethionine and/or selenocysteine, are stable in the solution for at least three months when stored at a temperature of between 1 and 50 °C.

3. The medical product according to any one of the preceding claims, wherein the selenate salt and/or a seleno-amino acid, such as selenomethionine and/or selenocysteine, are stable in the solution for at least 6 months, preferably 12 months, more preferably 18 months, most preferably 24 months at a temperature of from about 18°C to 25°C.

4. The medical product according to any one of the preceding claims, wherein the solution is a sterilized solution.

5. The medical product according to any one of the preceding claims, wherein the solution comprises a selenate salt, preferably selected from the group consisting of sodium selenate, potassium selenate, barium selenate and ammonium selenate, more preferably selected from sodium selenate and potassium selenate.

6. The medical product according to any one of the preceding claims, wherein the solution comprises at least one additional trace element, preferably selected from the group comprising zinc, iron, copper, manganese, chromium, iodine, fluoride and molybdenum, preferably zinc, iron, manganese and/or copper.

7. The medical product according to any one of the preceding claims, wherein the solution comprises an acid, preferably an organic acid selected from the group comprising malic acid, tartaric acid, citric acid, maleic acid, fumaric acid, more preferably malic acid, wherein the concentration of the organic acid is preferably in the range of from 50 mM to 400 mM, preferably from 190 mM to 220 mM, and more preferably about 200 mM.

8. The medical product according to any one of the preceding claims, wherein the solution comprises malic acid.

9. The medical product according to any one of the preceding claims, wherein the solution has an acidic pH, preferably in the range of from 1 to 4, more preferably from 2 to 3.5, more preferably from about 2.2 to 3.2.

10. The medical product according to any one of the preceding claims, wherein the solution does not comprise macronutrients selected from the group comprising carbohydrates, proteins and lipids, and wherein preferably the solution does not comprise any other nutrients.

11. The medical product according to any one of the preceding claims, wherein the solution is administered parenterally.

12. The medical product according to any one of the preceding claims, wherein the solution comprises a selenate salt and/or a seleno-amino acid, such as selenomethionine and/or selenocysteine, in an amount corresponding to 10-200 µg, preferably 40-100 µg, more preferably about 55 - 75 µg of selenium.

13. A medical product for preventing or correcting selenium deficiency in a patient according to any of the preceding claims, wherein the solution is contained in one chamber of a multi chamber container having at least two, at least three, at least four, at least five or at least six chambers.

14. The medical product according to claim 13, wherein the container comprises at least a first chamber containing a carbohydrate formulation, a second chamber containing an amino acid formulation, a third chamber containing a lipid formulation, optionally comprises electrolytes and/or vitamins in at least the first, second and/or third chamber, and wherein at least one chamber comprises a selenate salt and/or a seleno-amino acid, such as selenomethionine and/or selenocysteine.

15. The medical product according to claim 13 or 14, wherein the solution comprising a selenate salt and/or a seleno-amino acid, such as selenomethionine and/or selenocysteine, is present in a fourth chamber.

16. The medical product according to any one of claims 13 to 15, wherein the solution comprises sodium selenate or potassium selenate.

17. A method of preparing a medical product for preventing or correcting selenium deficiency in a patient according to any one of the preceding claims, wherein the solution is prepared by the steps comprising
a. dissolving a selenate salt and/or a seleno-amino acid, such as selenomethionine and/or selenocysteine, in a liquid medium, preferably water for injection, to produce a solution with a concentration of selenium between 0.28 and 28 mg/L,
b. optionally adjusting the solution to an acidic pH, preferably by providing an organic acid, such as malic acid, and optionally adding at least one additional trace element, preferably selected from the group comprising zinc, iron, copper, manganese, chromium, iodine, fluoride and molybdenum,
c. sterilizing the solution, preferably by heat sterilization.

18. A sterilized solution for parenteral administration comprising at least one compound selected from the group comprising a selenate salt and/or a seleno-amino acid, such as selenomethionine and/or selenocysteine, for use in preventing or correcting selenium deficiency in a patient.
